Europäisches Patentamt

European Patent Office

Office européen des brevets

(11) Publication number: 0 248 440
A2

# EUROPEAN PATENT APPLICATION

(21) Application number: 87108135.2

(51) Int. Cl.4: C07K 7/06 , A61K 37/02

(22) Date of filing: 04.06.87

Claims for the following Contracting States: AT + ES + GR.

(30) Priority: 06.06.86 US 871721

(43) Date of publication of application:
09.12.87 Bulletin 87/50

(84) Designated Contracting States:
AT BE CH DE ES FR GB GR IT LI LU NL SE

(71) Applicant: F. HOFFMANN-LA ROCHE & CO. Aktiengesellschaft
CH-4002 Basel(CH)

(72) Inventor: Danho, Waleed
118 Bunker Hill Road
Wayne, N.J. 07470(US)

(74) Representative: Lederer, Franz, Dr. et al
Patentanwält Dr. Franz Lederer Lucile
Grahnstrasse 22
D-8000 München 80(DE)

(54) Peptides.

(57) Peptides of the formula
$X-(R)_n-Tyr(SO_3H)-R^1-R^2-R^3-Met-R^4-(NCH_3-Phe)-Y$ I
wherein
X is $CO-R^5$, $CO(CH_2)_m-COOR^6$ or $COCOOR^7$
Y is $OR^8$ or $N(R^9,R^{10})$
n is 1 or 0; m is 1, 2 or 3
R is Asp, $NH_2C_2H_4$-Gly or palmitic acid
$R^1$ is Met, Nle, Leu or Ile
$R^2$ is Gly, Ala, $\beta$-Ala or (D) Ala
$R^3$ is Trp or Trp(CHO)
$R^4$ is Thr $(SO_3H)$, $Ser(SO_3H)$ or $Hyp(SO_3H)$
$R^5,R^6$ and $R^8$ to $R^{10}$ independently are hydrogen or $C_{1-3}$-alkyl
$R^7$ is hydrogen, $C_{1-3}$-alkyl or halo-$C_{1-3}$-alkyl,
and the pharmaceutically acceptable salts thereof, useful particularly for suppressing or reducing food intake and controlling appetite, can be prepared from corresponding unsulfated peptides.

EP 0 248 440 A2

## Peptides

The present invention is concerned with novel peptides, medicaments based thereon and a process for the manufacture of these peptides.

The peptides of the invention have the formula

$X\text{-}(R)_n\text{-}Tyr(SO_3H)\text{-}R^1\text{-}R^2\text{-}R^3\text{-}Met\text{-}R^4\text{-}(NCH_3\text{-}Phe)\text{-}Y$  I

wherein

$X$ is $CO\text{-}R^5$, $CO(CH_2)_m\text{-}COOR^6$ or $COCOOR^7$

$Y$ is $OR^8$ or $N(R^9,R^{10})$

$n$ is 1 or 0; $m$ is 1, 2 or 3

$R$ is Asp, $NH_2C_2H_4\text{-}Gly$ or palmitic acid

$R^1$ is Met, Nle, Leu or Ile

$R^2$ is Gly, Ala, $\beta$-Ala or (D) Ala

$R^3$ is Trp or Trp(CHO)

$R^4$ is Thr $(SO_3H)$, Ser($SO_3H$) or Hyp($SO_3H$)

$R^5,R^6$ and $R^8$ to $R^{10}$ independently are hydrogen or $C_{1-3}$-alkyl

$R^7$ is hydrogen, $C_{1-3}$-alkyl or halo-$C_{1-3}$-alkyl,

and the pharmaceutically acceptable salts thereof.

Amino acids are given their commonly understood three-letter designation herein. Unless otherwise specified, the L-isomer is meant.

Examples of salts of the peptides of formula I are alkaline or alkaline earth metal salts, e.g. the sodium, potassium or calcium salts, ammonium salts, alkylamine salts, e.g. di-or triethylamine salts, or acid addition salts, preferably hydrochlorides.

Preferred compounds of the inventions are those, wherein X is propionyl or especially acetyl (Ac) and Y is methylamino or especially amino, further those wherein $R^1$ is Met, $R^2$ is Gly and $R^4$ is Thr($SO_3H$) and those wherein n is 0. Particularly preferred are the peptides of the group consisting of

Ac-Tyr($SO_3H$)-Met-Gly-Trp(CHO)-Met-Thr($SO_3H$)-($NCH_3$-Phe)-$NH_2$,    Ac-Tyr($SO_3H$)-Nle-(D)Ala-Trp-Met-Thr-($SO_3H$)-($NCH_3$-Phe)-$NH_2$ Ac-($NH_2C_2H_4$-Gly)-Tyr($SO_3H$)-Met-Gly-Trp(CHO)-Met-Thr($SO_3H$)-($NCH_3$-Phe)-$NH_2$ and especially

Ac-Tyr($SO_3H$)-Met-Gly-Trp-Met-Thr($SO_3H$)-($NCH_3$-Phe)-$NH_2$.

The peptides of the invention may be prepared using solid phase synthesis, e.g. as described in J.A.C.S. 85 (1963) 2149. Solid-phase synthesis is commenced from the C-terminal end of the peptide by coupling a protected $\alpha$-amino acid by an amide bond to a suitable resin, e.g., benzylhydrylamine (BHA) or methylbenzylhydrylamine. As BHA resin there can be utilized a copolymer of styrene-1% divinylbenzene in bead form (200-400 mesh).

In solid phase synthesis, the reactive side chain groups of the various amino acid moieties are protected with suitable protecting groups which will prevent a chemical reaction from occurring at that site until the protecting group is ultimately removed. Examples of protecting groups for carboxyl groups are esters, such as aryl esters, e.g. phenyl esters optionally substituted with lower alkyl, halo, nitro, thio or $C_{1-7}$-alkylthio. The $\alpha$-amino groups can be blocked with t-butyloxycarbonyl (Boc) functions. The side chain groups in Asp, Ser, Thr can be blocked with benzyl, those in Trp with formyl, those in Tyr with 2,6-dichloro benzyl, those in amino ethyl glycine ($NH_2C_2H_4$-Gly) with benzyloxy carbonyl.

Boc-($NCH_3$-Phe) can be coupled to the BHA resin in the presence of dicyclohexylcarbodiimide (DCC) at 0°C, the loading being determined by amino acid analysis to be 0.20 mmol/g resin.

In the following protocol for a typical synthetic cycle EDT means 1,2-ethanedithiol, TFA: trifluoracetic acid, DIPEA: diisopropylethylamine, DMF: dimethylformamide.

| Step | Reagent | Time |
|------|---------|------|
| 1 | 1% EDT/$CH_2Cl_2$ | 30 sec |
| 2 | 50% TFA/$CH_2Cl_2$ w/1% EDT | 1 min |
| 3 | Repeat Step 1 | |
| 4 | 50% TFA/$CH_2Cl_2$ w/1% EDT | 15 min |
| 5 | $CH_2Cl_2$ | 30 sec |
| 6 | 2-Propanol | 30 sec |
| 7-8 | Repeat steps 5 & 6 | |
| 9 | $CH_2Cl_2$ | 2x30 sec |
| 10 | 8% DIPEA | 2x 2 min |
| 11-15 | Repeat step 5-9 | |
| 16 | 5 equiv. of Boc-amino acid anhydride | 30 min |
| 17 | 1% DIPEA | 5 min |
| 18-19 | Repeat steps 6 & 9 | |
| 20-21 | Repeat steps 16 & 17 if coupling was complete after step 19 | |
| 22 | 2-Propanol | 30 sec |
| 23-24 | Repeat steps 5 & 6 | |
| 25 | $CH_2Cl_2$ | 30 sec |
| 26 | DMF | 2x30 sec |
| 27 | $CH_2Cl_2$ | 3x30 sec |

Solvents for all washings and couplings are measured to volumes of 10-20 ml/g resin. Couplings are performed with the symmetrical anhydrides of the Boc-amino acids. They are performed in $CH_2Cl_2$ at 0°C in 15 min using 10 equivalents of Boc-amino acid and 5 equivalents of DCC. Total cycle times ranges from 54-160 minutes per residue.

Deblocking and cleavage of the fully assembled peptide-resins can be performed with HF, dimethylsulfide and p-cresol at 0°C. The unsulfated peptides thus obtained can be purified by preparative HPLC.

The peptides of the invention can be prepared by a process comprising reacting the corresponding unsulfated peptides with a sulfating agent, or reacting a formylated peptide of formula I with a deformylating agent, and if desired, isolating an obtained peptide of formula I in form of a salt.

As sulfating agent there can be utilized pyridinum acetyl sulfate (PAS). The reaction can be conducted at 60°C. The obtained sulfated peptides of formula I can be purified by preparative reverse phase HPLC.

The formyl group present in a peptide of the invention, can be cleaved using 0.1N $NH_4OH$ at room temperature followed by lyophilization.

Unlike CCK-8 [Life Sciences 30 (1982) p479:485-488], which binds to receptor sites in the pancreas and brain, the peptides of the invention bind primarily to receptor sites in the pancreas. Further, the peptides of the invention are more resistant to degradation by various gastrointestinal peptidase enzymes than is CCK-8. As a result, the peptides of the invention are longer acting than CCK-8.

The following assay illustrates the specific binding of the peptides of the invention to receptors in homogenized preparations of rat pancreatic cells and bovine striata compared to the binding of native CCK-8 using $^3$H-CCK-8-($SO_3$H) as a radioligand.

About 5 g of frozen bovine striatum and about 5 g of fresh rat pancreas were homogenized in a buffer (pH 7.4). The tissue was isolated by centrifugation at 48,000 x g for 10 min at 0°C and then resuspended: 1 part striatal tissue per 80 parts buffer and 1 part pancreas tissue per 70 parts buffer. Incubation was initiated by combining various concentrations of CCK-8 or peptides of formula I with $^3$H-CCK-8-($SO_3$H) (final conc. = 0.15 nM) and tissue homogenate (striatum ~ 0.26 mg protein in 2 ml final volume; pancreas ~ 0.165 mg

3

protein in 1 ml final volume). Samples were incubated for 30 min at 25°C and the incubation terminated by filtering the mixture. The filter was air dried and then placed in a scintillation vial with 12 ml of a scintillation cocktail. The vials were shaken for 2 hours and then counted using a scintillation spectrometer. Non-specific binding was determined in the presence of 1 μm CCK-8 and subtracted from all samples to determine specific binding. The concentration of peptide necessary to inhibit 50% of total specific $^3$H-CCK-8-(SO$_3$H) binding (IC$_{50}$ value) was determined. The results are summarized in Table 1.

In a two meal feeding assay, male rats weighing 180-200 g were fasted for two days, weighed, placed in individual cages and a four-day period or meal training was begun. During this time the rats were given chow in cups from 9:00 until 10:00 a.m., the cups were removed from 10:00 a.m. to 12.00 p.m. and placed back in the cages from 12:00 until 1:00 p.m.. On the fourth day, the rats were weighed again and any which lost more than five grams body weight were excluded from the test. The animals were then distributed into experimental and control groups. Peptides of the invention were suspended either in saline, if soluble, or in 1% gum arabic, if insoluble, at concentrations of 0 to 32 μg/ml/kg body weight and were administered intraperitoneally 15 min before the first meal on day 5 of meal feeding. The rats were then given their meals as they had been during the previous four days and the food cups were weighed both before and after each meal to determine food consumption. Food intake for the treated groups were compared to that of the control groups. The results are summarized in Table 1.

## Table 1

| Peptide of Example No. | Bovine Striatum (nM) | Rat Pancreas (nM) | Food Intake % 1st Meal | 2nd Meal |
|---|---|---|---|---|
| 1 | 1000 | 9.4 | 23±13 | 55±10 |
| 2 | 100,500 | 2.2 | 8±1 | 26±4 |
| 3 | 100 | 100 | 34±6 | 164±16 |
| 4 | 100 | 44 | 5±5 | 79±13 |
| 5 | 4100 | 4200 | 70±2 | 133±9 |
| 6 | 1750 | 100 | 14±6 | 114±9 |
| 7 | 1000 | 14.5 | 17 | 95 |
| CCK-8 | 1-4,6 | 1-32 | 27±17 | 149±6 |

The following assay illustrates the satiety inducing effect of the peptide of Example 2:

Male rats were meal trained as described above. The peptide of Example 2 was dissolved in saline and administered to the rats intranasally. CCK-8, dissolved in saline was used as a control. The results are summarized in Table 2.

## Table 2

### Food Intake in grams consumed
### (and in %)

| Treatment | Dose mg/kg | 1st meal | 2nd meal |
|-----------|------------|----------|----------|
| Saline | | 7.4 ± 0.5 (100) | 5.6 ± 0.3 (100) |
| Peptide of | 3 | 7.5 ± 0.8 (102) | 6.1 ± 0.3 (109) |
| Example 2 | 10 | 5.5 ± 0.6 (74) | 6.3 ± 0.4 (112) |
| | 30 | 5.8 ± 0.7 (78) | 4.3 ± 0.6 (76) |
| | 100 | 3.4 ± 0.4 (46) | 4.6 ± 0.5 (82) |
| CCK-8 | 1000 | 4.9 ± 0.7 (66) | 6.3 ± 0.5 (112) |
| | 400 | inactive | |

As illustrated below the peptides of the invention are more resistant than CCK-8 to degradation by enzymes: Rat kidney metalloendopeptidase (0.01 $\mu$g) and 5 nmol of peptide were incubated in 2 $\mu$mol tris-HCl buffer (pH 7.6) and at 37°C for varying periods of time. The degradation of the peptide was monitored by analytical HPLC and is summarized in Table 3. Rates are based on presumed production of Asp-Tyr-(SO$_3$H)-Met-Gly for CCK-8 and the presumed production of Ac-Tyr (SO$_3$H)-Met-Gly for the peptides of Example 2 and 3.

Table 3

| Peptide of Example No. | Time of incubation(min) | %Degradation by height | No. of peaks |
|---|---|---|---|
| 2 | 0 | 0 | |
| | 60 | 19.4 | |
| | 120 | 30.3 | |
| | 240 | 56.4 | 2* |
| 3 | 0 | 0 | |
| | 60 | 0 | |
| | 120 | 0 | |
| | 240 | 0 | 0 |
| CCK-8 | 0 | 0 | |
| | 15 | 6.2 | |
| | 30 | 33.2 | |
| | 60 | 69.7 | 4 |

*   Indicates one peak detected and one peak that co-eluted with substrate that was differentiated based on molar ratios following amino acid analysis.

The peptides of formula I and the pharmaceutically acceptable salts thereof are useful for suppressing or reducing food intake in humans or animals and may thus be used for controlling or reducing the body weight. They may be administered intravenously, sublingually, rectally, intranasally, buccally or transdermally.

The pharmaceutical or veterinary compositions containing a peptide of the invention and/or a salt thereof may be prepared in a conventional way and can contain conventional carriers and/or diluents. When formulated for injection, peptides of the invention and salts thereof are preferably presented as sterile powder to be reconstituted with water for injections or other suitable sterile vehicle shortly before administration. For example, for intravenous injections, sterile aqueous isotonic solutions may be used, preferably sodium chloride isotonic aqueous solution. The sterile powder is conveniently obtained by means of lyophilization; in that case the active ingredient is conveniently admixed with an inert carrier, such as lactose.

Example 1

Boc-(NCH₃-Phe) (5 g 17.8 mmol) was dissolved in a mixture of 50 ml of $CH_2Cl_2$, 50 ml of DMF chilled to 0°C and 1.8 g (9 mmol) of DCC were added and the mixture was stirred for 60 minutes at 0°C. 5 g of BHA resin (copolymer of styrene-1% divinylbenzene) having a total nitrogen content of 0.56 mmol/g, was washed with 10% DIPEA in $CH_2Cl_2$ for 30 min, filtered and washed with $CH_2Cl_2$, DMF and $CH_2Cl_2$. The chilled mixture above was added to the resin and stirred for 24 hours at room temperature. The resin was filtered and washed with $CH_2Cl_2$, DMF and isopropanol and dried under high vacuum.

Amino acid analysis showed the resin to contain 0.20 mmoles/g of Boc-(NCH₃-Phe). Unreacted amine groups were capped by shaking the resin with 5 ml of acetic anhydride and 5 ml of DIPEA in $CH_2Cl_2$ for 60 minutes. The resin was filtered and washed with $CH_2Cl_2$, isopropanol and DMF.

4.8 g (0.96 mmole) of the Boc-(NCH₃-Phe) containing resin were subjected to sequential solid phase synthesis using the procedure described above, all couplings being performed using the symmetrical anhydrides of Boc-amino acids. At step 16 and 20 the activated amino acids were added with the corresponding reaction times as follows: six individual cycles were performed with Boc-O-benzyl-Thr (1.5 g, 5 mmole, 60 min, twice), Boc-Met (1.25 g, 5 mmole, 30 min, twice), Boc-N$^{in}$-Trp(CHO) (1.7 g, 5 mmole, 30 min, twice), Boc-Gly (900 mg, 5 mmole, 3 min, twice), Boc-Met (1.25 g, 5 mmole 30 min, twice) and Boc-2,6-dichlorobenzyl-Tyr (2.2 g, 5 mmole, 30 min, twice).

Deprotection of the Boc-protecting group and acetylation of the resin with 20 ml acetic anhydride, 20 ml of pyridine in $CH_2Cl_2$ for 60 min yielded 5.3 g of the acetylated heptapeptide containing resin. These were cleaved by treatment with 12 ml of HF, 31 ml of dimethylsulfide and 4.6 ml of p-cresol for 1 hour at 0°C. After evaporation, 42 ml of anhydrous HF were distilled into the reaction vessel for a second treatment for 2 h at 0°C. After thorough evaporation, the resin was washed with 2 volumes of ethyl acetate, then triturated with 4 x 15 ml of 30% acetic acid and filtered lyophilized to yield 400 mg of crude peptide.

140 mg of the crude peptide was purified by preparative HPLC on a (2.5 x 50) cm micronized silica gel column, eluting with TFA/CH₃CN (5 to 65%/0.022%) at a flow rate of 8 ml/min. Detection was at 280 nm. The main peak was collected and lyophilized to yield 55 mg (16.1%) of unsulfated peptide.

To 60 mg of PAS were added 10 ml of dry distilled pyridine. The resulting mixture was heataed at 60°C, with stirring for 10 min. The solution was allowed to cool and 10 mg of Ac-Tyr-Met-Gly-Trp(CHO)-Met-Thr-(NCH₃-Phe)-NH₂ dissolved in 10 ml of pyridine were added to the solution. The reaction mixture was stirred for 1 hour at 60°C. Then, the reaction mixture was neutralized with 2 volumes of 0.05M ammonium bicarbonate and lyophilized. Purification was achieved by preparative reverse phase HPLC on octadecyl silica with particle diameter of 10μ, eluting with 0.05M NH₄HCO₃/CH₃CN. The yield was 5 mg (43%) of Ac-Tyr($SO_3H$)-Met-Gly-Trp(CHO)-Met-Thr($SO_3H$) -(NCH₃-Phe)-NH₂.

## Example 2

2 mg of the product of Example 1 were dissolved in 2.0 ml of 0.1N NH₄OH (pH 10.5) and allowed to stand for 1 hour at room temperature. The solution was then lyophilized to yield 1.7 mg (90%) of Ac-Tyr-($SO_3H$)-Met-Gly-Trp-Met-Thr($SO_3H$)-(NCH₃-Phe)-NH₂.

## Example 3

4.6 g (0.9 mmole) of Boc-(NCH₃-Phe) containing resin were prepared and subjected to sequential solid phase synthesis using the same procedure as in Example 1. At step 16 and 20 of the synthesis, the activated amino acids were added with the corresponding reaction times as follows: six individual cycles were performed with Boc-O-benzyl-Thr (1.5 g, 5 mmole, 60 min, twice, Boc-Met (1.25 g, 5 mmole, 30 min, twice), Boc-N$^{in}$-Trp(CHO) (1.7 g, 5 mmole, 30 min, twice), Boc-(D)-Ala (950 mg, 5 mmole, 30 min), Boc-Nle (1.15 g, 5 mmole, 60 min) and Boc-2,6-dichlorobenzyl-Tyr (2.2 g, 5 mmole, 30 min, twice).

Deprotection of the Boc-protecting group and acetylation of the resin as in example 1 yielded 5.2 g of the acetylated heptapeptide resin. This was cleaved by treatment with HF containing dimethylsulfide and p-cresol using the procedure of Example 1. The resin was washed with diethyl ether and ethyl acetate, then triturated with 4 x 15 ml of 30% acetic acid, filtered and lyophilized to yield 80 mg of crude peptide. This was purified by preparative HPLC analogously to Example 1 to yield 50 mg (5.36%) of the unsulfated peptide.

10 mg of Ac-Tyr-Nle-(D)Ala-Trp(CHO)-Met-Thr-(NCH₃-Phe)-NH₂ were reacted with PAS and then purified by preparative HPLC as described in Example 1 to yield 8 mg (70%) of Ac-Tyr($SO_3H$)-Nle-(D)Ala-Trp-(CHO)-Met-Thr($SO_3H$) -(NCH₃-Phe)-NH₂.

## Example 4

2 mg of the product of Example 3 were treated analogously to Example 2 to yield 1.8 mg (93%) of Ac-Tyr($SO_3H$)-Nle-(D)Ala-Trp-Met-Thr($SO_3H$) -(NH₃-Phe)-NH₂.

Example 5

2.4 g (0.384 mmole) of Boc-(NCH₃-Phe) resin, obtained in the same way as in Example 1, were subjected to sequential solid phase synthesis as in Example 1. At step 16 and 20 the activated amino acids were added with the corresponding reactions times as follows: six individual cycles were performed with Boc-O-benzyl-Thr (1.5 g, 5 mmol, 60 min; 1,5 g, 5 mmole, 30 min), Boc-Met (1.25 g, 5 mmole, 30 min, twice), Boc Nⁱⁿ-Trp(CHO) (1.7 g, 5 mmole, 30 min, twice), Boc-Ala (0.95 g, 5 mmole, 30 min), Boc-Met (1.25 g, 5 mmole, 30 min, twice) and Boc-2,6-dichlorobenzyl-Tyr (2.2 g, 5 mmole, 30 min, twice).

Deprotection of the Boc-protecting group and acetylation of the resin as in Example 1 yielded 3.0 g of acetylated heptapeptide resin. The resin was cleaved and then washed, triturated with acetic acid, filtered and lyophilized to yield 100 g of crude peptide. This was purified by preparative HPLC as in Example 1 to yield 25 mg (6.6%) of unsulfated peptide.

12 mg of Ac-Tyr-Met-Ala-Trp(CHO)-Met-Thr-(NCH₃-Phe)-NH₂ were treated with PAS and then purified as described in Example 1 to yield 9.8 mg (70%) of Ac-Tyr(SO₃H)-Met-Ala-Trp(CHO)-Met-Thr(SO₃H) -- (NCH₃-Phe)-NH₂.

Example 6

2 mg of the product of Example 5 were treated as in Example 2 to yield 1.9 mg (95%) of Ac-Tyr(SO₃H)-Met-Ala-Trp-Met-Thr(SO₃H) -(NCH₃-Phe)-NH₂.

Example 7

4.4 g (0.9 mmole) of Boc-(NCH₃-Phe) resin, obtained as in Example 1, were subjected to sequential solid phase synthesis as in Example 1. At step 16 and 20, the activated amino acids were added with the corresponding reactions times as follows: seven individual cycles were performed with O-benzyl-Thr (1.5 g, 5 mmole, 60 min, twice), Boc-Met (1.25 g 5 mmole, 30 min, twice), Boc-Nⁱⁿ -Trp(CHO) (1.7 g, 5 mmole, 30 min, twice), Boc-Gly (900 mg, 5 mmole, 30 min, twice), Boc-Met (1.25 g, 5 mmole, 30 min, twice), Boc-2,6-dichlorobenzyl-Tyr (2.2 g, 5 mmole, 30 min, twice), Boc-(NH₂C₂H₄-Gly)-OH (3.5 g, 5 mmol, 60 min, 3.5 g, 5 mmole, 30 min).

Deprotection of the Boc-protecting group and acetylation of the resin as in example 1 yielded 5.6 g of the acetylated-octapeptide resin. This was cleaved, then washed with ethyl acetate and triturated with acetic acid, filtered and lyophilized as in Example 1 to yield 582 mg of crude peptide.

75 mg of the crude peptide were purified by preparative HPLC as in Example 1 to yield 8 mg (6.2%) of unsulfated peptide,
Ac-(NH₂C₂H₄-Gly)-Tyr-Met-Gly-Trp(CHO)-Met-Thr-(NCH₃-Phe)-NH₂.

This was treated as in Example 1 to yield 45 mg (45%) of Ac-(NH₂C₂H₄-Gly)-Tyr(SO₃H)-Met-Gly-Trp-(CHO)-Met-Thr-(SO₃H)-(NCH₃-Phe)-NH₂.

**Claims**

1. Peptides of the formula
X-(R)$_n$-Tyr(SO₃H)-R¹-R²-R³-Met-R⁴-(NCH₃-Phe)-Y I
wherein
X is CO-R⁵, CO(CH₂)$_m$-COOR⁶ or COCOOR⁷
Y is OR⁸ or N(R⁹,R¹⁰)
n is 1 or 0; m is 1, 2 or 3
R is Asp, NH₂C₂H₄-Gly or palmitic acid
R¹ is Met, Nle, Leu or Ile
R² is Gly, Ala, β-Ala or (D) Ala
R³ is Trp or Trp(CHO)
R⁴ is Thr (SO₃H), Ser(SO₃H) or Hyp(SO₃H)
R⁵,R⁶ and R⁸ to R¹⁰ independently are hydrogen or C$_{1-3}$-alkyl
R⁷ is hydrogen, C$_{1-3}$-alkyl or halo-C$_{1-3}$-alkyl,
and the pharmaceutically acceptable salts thereof.

2. Peptides according to claim 1 wherein X is propionyl or especially acetyl and Y is methylamino or especially amino.

3. Peptides according to claim 1 or 2 wherein $R^1$ is Met, $R^2$ is Gly and $R^4$ is Thr(SO$_3$H).

4. Peptides according to claim 1, 2 or 3 wherein n is 0.

5. Peptides according to claim 1 of the group consisting of Ac-Tyr(SO$_3$H)-Met-Gly-Trp(CHO)-Met-Thr-(SO$_3$H)-(NCH$_3$-Phe)-NH$_2$, Ac-Tyr(SO$_3$H)-Nle-(D)Ala-Trp-Met-Thr(SO$_3$H)-(NCH$_3$-Phe)-NH$_2$ Ac-(NH$_2$C$_2$H$_4$-Gly)-Tyr(SO$_3$H)-Met-Gly-Trp(CHO)-Met-Thr(SO$_3$H)-(NCH$_3$-Phe)-NH$_2$ and especially Ac-Tyr(SO$_3$H)-Met-Gly-Trp-Met-Thr(SO$_3$H)-(NCH$_3$-Phe)-NH$_2$.

6. The peptides of any one of claims 1 to 5 for use as therapeutically active substances, particularly for suppressing or reducing food intake and controlling appetite.

7. Pharmaceutical preparation based on a peptide of any one of claims 1 to 5, particularly for suppressing or reducing food intake and controlling appetite.

8. The use of a peptide of any one of claims 1 to 5 in the manufacture of a pharmaceutical preparation according to claim 7.

9. Process for the manufacture of the peptides of any one of claims 1 to 5, which comprises reacting the corresponding unsulfated peptides with a sulfating agent, or reacting a formylated peptide of formula I with a deformylating agent, and if desired, isolating an obtained peptide of formula I in form of a salt.

Claims for the following contracting states: AT, ES and GR

1. Process for the preparation of peptides of the formula
X-(R)$_n$-Tyr(SO$_3$H)-R$^1$-R$^2$-R$^3$-Met-R$^4$-(NCH$_3$-Phe)-Y I
wherein
X is CO-R$^5$, CO(CH$_2$)$_m$-COOR$^6$ or COCOOR$^7$
Y is OR$^8$ or N(R$^9$,R$^{10}$)
n is 1 or 0; m is 1, 2 or 3
R is Asp, NH$_2$C$_2$H$_4$-Gly or palmitic acid
R$^1$ is Met, Nle, Leu or Ile
R$^2$ is Gly, Ala, $\beta$-Ala or (D) Ala
R$^3$ is Trp or Trp(CHO)
R$^4$ is Thr (SO$_3$H), Ser(SO$_3$H) or Hyp(SO$_3$H)
R$^5$,R$^6$ and R$^8$ to R$^{10}$ independently are hydrogen or C$_{1-3}$-alkyl
R$^7$ is hydrogen, C$_{1-3}$-alkyl or halo-C$_{1-3}$-alkyl,
and the pharmaceutically acceptable salts thereof, which process comprises reacting the corresponding unsulfated peptides with a sulfating agent, or reacting a formylated peptide of formula I with a deformylating agent, and if desired, isolating an obtained peptide of formula I in form of a salt.

2. Process according to claim 1 wherein X is propionyl or especially acetyl and Y is methylamino or especially amino.

3. Process according to claim 1 or 2 wherein $R^1$ is Met, $R^2$ is Gly and $R^4$ is Thr(SO$_3$H).

4. Process according to claim 1, 2 or 3 wherein n is 0.

5. Process according to claim 1, wherein a peptide of the group consisting of Ac-Tyr(SO$_2$H)-Met-Gly-Trp(CHO)-Met-Thr(SO$_3$H)-(NCH$_3$-Phe)-NH$_2$, Ac-Tyr(SO$_3$H)-Nle-(D)Ala-Trp-Met-Thr(SO$_3$H)-(NCH$_3$-Phe)-NH$_2$ Ac-(NH$_2$C$_2$H$_4$-Gly)-Tyr(SO$_3$H)-Met-Gly-Trp(CHO)-Met-Thr(SO$_3$H)-(NCH$_3$-Phe)-NH$_2$ and especially Ac-Tyr(SO$_3$H)-Met-Gly-Trp-Met-Thr(SO$_3$H)-(NCH$_3$-Phe)-NH$_2$ is prepared.

6. Process for the manufactue of a pharmaceutical preparation, particularly for suppressing or reducing food intake and controlling appetite, which process comprises bringing a compound of formula I in claim 1 or a pharmaceutically acceptable salt thereof in a galenical form.

7. Pharmaceutical preparation, particularly for suppressing or reducing food intake and controlling appetite, which preparation contains a compound of formula I in claim 1 or a pharmaceutically acceptable salt thereof.

8. The use of a peptide of any one of claims 1 to 5 in the manufacture of a pharmaceutical preparation according to claim 7.

9. Peptides of the formula
X-(R)$_n$-Tyr(SO$_3$H)-R$^1$-R$^2$-R$^3$-Met-R$^4$-(NCH$_3$-Phe)-Y I
wherein
X is CO-R$^5$, CO(CH$_2$)$_m$-COOR$^6$ or COCOOR$^7$
Y is OR$^8$ or N(R$^9$, R$^{10}$) .
n is 1 or 0; m is 1, 2 or 3

R is Asp, $NH_2C_2H_4$-Gly or palmitic acid

$R^1$ is Met, Nle, Leu or Ile

$R^2$ is Gly, Ala, $\beta$-Ala or (D) Ala

$R^3$ is Trp or Trp(CHO)

$R^4$ is Thr $(SO_3H)$, Ser$(SO_3H)$ or Hyp$(SO_3H)$

$R^5,R^6$ and $R^8$ to $R^{10}$ independently are hydrogen or $C_{1-3}$-alkyl

$R^7$ is hydrogen, $C_{1-3}$-alkyl or halo-$C_{1-3}$-alkyl,

and the pharmaceutically acceptable salts thereof, whenever prepared by the process of claim 1 or by an obvious chemical equivalent thereof.

10. Peptides according to claim 9 wherein X is propionyl or especially acetyl and Y is methylamino or especially amino, whenever prepared by the process of claim 2 or by an obvious chemical equivalent thereof.

11. Peptides according to claim 9 or 10 wherein $R^1$ is Met, $R^2$ is Gly and $R^4$ is Thr$(SO_3H)$, whenever prepared by the process of claim 3 or by an obvious chemical equivalent thereof.

12. Peptides according to claim 9, 10 or 11 wherein n is 0, whenever prepared by the process of claim 4 or by an obvious chemical equivalent thereof.

13. Peptides according to claim 9 of the group consisting of Ac-Tyr$(SO_3H)$-Met-Gly-Trp(CHO)-Met-Thr-$(SO_3H)$-$(NCH_3$-Phe)-$NH_2$, Ac-Tyr$(SO_3H)$-Nle-(D)Ala-Trp-Met-Thr$(SO_3H)$-$(NCH_3$-Phe)-$NH_2$ Ac-$(NH_2C_2H_4$-Gly)-Tyr$(SO_3H)$-Met-Gly-Trp(CHO)-Met-Thr$(SO_3H)$-$(NCH_3$-Phe)-$NH_2$ and especially Ac-Tyr$(SO_3H)$-Met-Gly-Trp-Met-Thr$(SO_3H)$-$(NCH_3$-Phe)-$NH_2$, whenever prepared by the process of claim 5 or by an obvious chemical equivalent thereof.

14. The invention as herein described, particularly with reference to the Examples.